Europäisches Patentamt

European Patent Office

Office européen des brevets

(19)

(11) **EP 0 950 197 B1**

(12) **FASCICULE DE BREVET EUROPEEN**

(45) Date de publication et mention
de la délivrance du brevet:
**02.05.2003 Bulletin 2003/18**

(51) Int Cl.⁷: **G01T 1/164**

(21) Numéro de dépôt: **97953959.0**

(22) Date de dépôt: **29.12.1997**

(86) Numéro de dépôt international:
**PCT/FR97/02436**

(87) Numéro de publication internationale:
**WO 98/029763 (09.07.1998 Gazette 1998/27)**

(54) **DISPOSITIF ET PROCEDE DE LOCALISATION NUCLEAIRE PAR CALCUL DE BARYCENTRE ITERATIF, ET APPLICATION AUX GAMMA-CAMERAS**

VERFAHREN UND VORRICHTUNG ZUR KERNSTRAHLUNGSLOKALISIERUNG MITTELS ITERATIVER SCHWERPUNKBESTIMMUNG FÜR EINER GAMMAKAMERA

DEVICE AND METHOD FOR NUCLEAR LOCATING BY ITERATIVE COMPUTING OF BARYCENTER, AND APPLICATION TO GAMMA-CAMERAS

(84) Etats contractants désignés:
**DE GB IT NL**

(30) Priorité: **31.12.1996 FR 9616295**

(43) Date de publication de la demande:
**20.10.1999 Bulletin 1999/42**

(73) Titulaire: **COMMISSARIAT A L'ENERGIE ATOMIQUE**
**75015 Paris (FR)**

(72) Inventeurs:
• **CHAPUIS, Alain**
**F-38950 Saint-Martin-le-Vinoux (FR)**
• **JANIN, Claude**
**F-38000 Grenoble (FR)**
• **TARARINE, Michel**
**F-92330 Sceaux (FR)**

(74) Mandataire: **Audier, Philippe André et al**
**Brevalex,**
**3, rue du Docteur Lancereaux**
**75008 Paris (FR)**

(56) Documents cités:
**EP-A- 0 252 566       EP-A- 0 634 672**
**US-A- 4 060 730        US-A- 4 611 283**
**US-A- 5 576 547**

**Description**

Domaine technique

La présente invention concerne un dispositif de détermination de la position d'un événement induisant un signal dans des photodétecteurs, cette position étant, par exemple, repérée par rapport à l'ensemble des photodétecieurs. Une telle position peut être repérée par le barycentre de l'événement dans un repère lié aux photodétecteurs.

L'invention s'applique en particulier à la détermination de la position d'un événement à partir de signaux fournis par des photomultiplicateurs équipant une gamma-caméra, la position étant repérée par rapport aux photomultiplicateurs eux-mêmes. On entend par gamma-caméra une caméra sensible au rayonnement gamma ($\gamma$). De telles caméras sont utilisées notamment à des fins d'imagerie médicale.

Etat de la technique antérieure

A l'heure actuelle, la plupart des gamma-caméras utilisées en médecine nucléaire sont des caméras fonctionnant selon le principe des caméras de type Anger. On peut se reporter à ce sujet au document US-3 011 057.

Les gamma-caméras permettent en particulier de visualiser la répartition, dans un organe, de molécules marquées par un isotope radioactif préalablement injecté au patient.

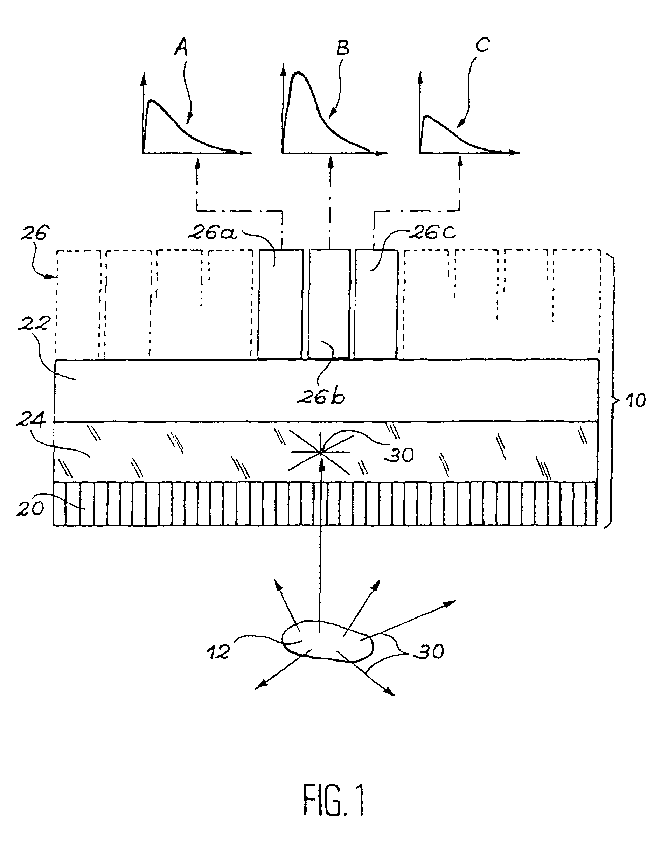

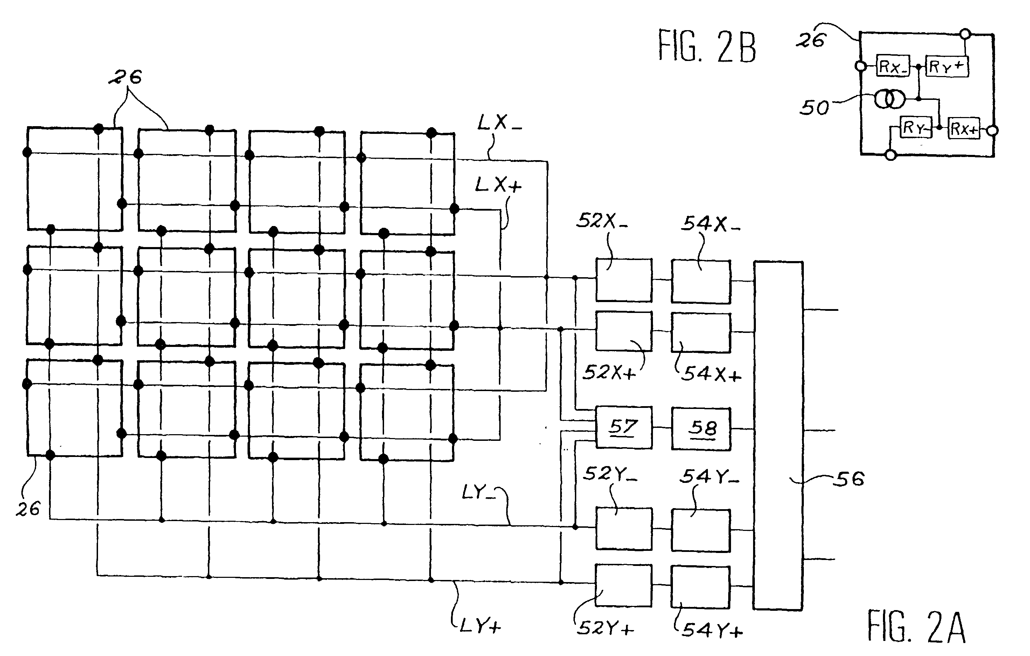

La structure et le fonctionnement d'une gamma-caméra connue sont exposés et résumés ci-après en référence aux figures 1, 2A et 2B annexées.

La figure 1 montre une tête de détection 10 d'une gamma-caméra disposée en face d'un organe 12 contenant des molécules marquées par un isotope radioactif.

La tête de détection 10 comporte un collimateur 20, un cristal scintillateur 22, un guide de lumière 24 et une pluralité de tubes photomultiplicateurs 26 juxtaposés de façon à couvrir une face du guide de lumière 24. Le scintillateur est, par exemple, un cristal de NaI(Tℓ).

Le collimateur 20 a pour fonction de sélectionner parmi tous les rayonnements gamma 30 émis par l'organe 12 ceux qui atteignent la tête de détection sensiblement sous incidence normale. Le caractère sélectif du collimateur permet d'augmenter la résolution et la netteté de l'image produite. Cependant, l'augmentation de la résolution se fait au détriment de la sensibilité. A titre d'exemple, pour environ 10000 photons $\gamma$ émis par l'organe 12, un seul photon est effectivement détecté.

Les photons $\gamma$ ayant traversé le collimateur atteignent le cristal scintillateur 22 où quasiment chaque photon $\gamma$ est converti en une pluralité de photons lumineux. Dans la suite du texte on désigne par événement chaque interaction d'un photon gamma avec le cristal, provoquant une scintillation.

Les photomultiplicateurs 26 sont conçus pour émettre une impulsion électrique proportionnelle au nombre de photons lumineux reçus du scintillateur pour chaque événement.

Pour qu'un événement de scintillation puisse être localisé plus précisément, les photomultiplicateurs 26 ne sont pas directement accolés au cristal scintillateur 22 mais sont séparés de ce dernier par le guide de lumière 24.

Les photomultiplicateurs émettent un signal dont l'amplitude est proportionnelle à la quantité totale de lumière produite dans le scintillateur par un rayonnement gamma, c'est-à-dire proportionnelle à son énergie. Toutefois, le signal individuel de chaque photomultiplicateur dépend aussi de la distance qui le sépare du point d'interaction 30 du rayonnement gamma avec la matière du scintillateur. En effet, chaque photomultiplicateur délivre une impulsion de courant proportionnelle au flux lumineux qu'il a reçu. Dans l'exemple de la figure 1, des petits graphiques A, B, C montrent que des photomultiplicateurs 26a, 26b et 26c situés à différentes distances d'un point d'interaction 30 délivrent des signaux avec des amplitudes différentes.

La position du point d'interaction 30 d'un photon gamma est calculée dans la gamma-caméra à partir des signaux provenant de l'ensemble des photomultiplicateurs en effectuant une pondération barycentrique des contributions de chaque photomultiplicateur.

Le principe de la pondération barycentrique tel qu'il est mis en oeuvre dans les caméras de type Anger apparaît plus clairement en se reportant aux figures 2A et 2B annexées.

La figure 2A montre le câblage électrique d'une tête de détection 10 d'une gamma-caméra, qui relie cette caméra à une unité de formation d'une image. La tête de détection comporte une pluralité de photomultiplicateurs 26.

Comme le montre la figure 2B, chaque photomultiplicateur 26 de la tête de détection est associé à quatre résistances notées $RX^-$, $RX^+$, $RY^-$ et $RY^+$. Les valeurs de ces résistances sont propres à chaque photomultiplicateur et dépendent de la position du photomultiplicateur dans la tête de détection 10.

Les résistances $RX^-$, $RX^+$, $RY^-$ et $RY^+$ de chaque photomultiplicateur sont reliées à la sortie 50 dudit photomultiplicateur, représentée sur la figure 2B avec un symbole de générateur de courant. Elles sont d'autre part respectivement reliées à des lignes collectrices communes notées $LX^-$, $LX^+$, $LY^-$, $LY^+$, sur la figure 2A.

Les lignes $LX^-$, $LX^+$, $LY^-$ et $LY^+$ sont à leur tour reliées respectivement à des intégrateurs analogiques $52X^-$,

$52X^+$, $52Y^-$, $52Y^+$, et, par l'intermédiaire de ceux-ci à des convertisseurs analogiques/numériques $54X^-$, $54X^+$, $54Y^-$, $54Y^+$. La sortie des convertisseurs $54X^-$, $54X^+$, $54Y^-$, $54Y^+$ est dirigée vers un opérateur numérique 56. Les lignes $LX^-$, $LX^+$, $LY^-$, $LY^+$ sont par ailleurs reliées à une voie commune, dite voie énergie. Cette voie comporte également un intégrateur 57 et un convertisseur analogique/numérique 58 et sa sortie est aussi dirigée vers l'opérateur 56.

Grâce au dispositif de la figure 2, on calcule la position de l'interaction selon les équations suivantes (US-4 672 542) :

$$X = \frac{X^+ - X^-}{X^+ + X^-}$$

et

$$Y = \frac{Y^+ - Y^-}{Y^+ + Y^-}$$

dans lesquelles X et Y indiquent les coordonnées selon deux directions orthogonales de la position de l'interaction sur le cristal et dans lesquelles $X^+$, $X^-$, $y^+$, $Y^-$ indiquent respectivement les signaux pondérés délivrés par les intégrateurs $52X^+$, $52X^-$, $52Y^+$, $52Y^-$.

Les valeurs de X et Y de même que l'énergie totale E du rayon gamma ayant interagi avec le cristal sont établies par l'opérateur numérique 56. Ces valeurs sont utilisées ensuite pour la construction d'une image comme décrit, par exemple, dans le document FR-2 669 439.

Le calcul de la position de l'interaction est entaché d'une incertitude liée aux fluctuations statistiques de Poisson du nombre de photons lumineux et du nombre de photoélectrons produits pour chaque événement, c'est-à-dire pour chaque photon gamma détecté. L'écart type de la fluctuation est d'autant plus faible que le nombre de photons ou de photoélectrons est élevé. En raison de ce phénomène, il convient de collecter la lumière le plus soigneusement possible. La résolution spatiale intrinsèque de la caméra est caractérisée par la largeur à mi-hauteur de la distribution des positions calculées pour une même source ponctuelle collimatée posée sur le cristal scintillateur.

Pour des rayons gamma d'une énergie de 140 keV, la résolution est généralement de l'ordre de 3 à 4 mm.

L'énergie d'un photon gamma détecté est calculée en faisant la somme des contributions de tous les photomultiplicateurs ayant reçu de la lumière. Elle est aussi entachée d'une fluctuation statistique. La résolution en énergie de la caméra est caractérisée par le rapport de la largeur à mi-hauteur de la distribution des énergies calculées à la valeur moyenne de la distribution, pour une même source.

La résolution en énergie est généralement de l'ordre de 9 à 11% pour des rayons gamma d'une énergie de 140 keV.

Finalement, une gamma-caméra de type Anger présente l'avantage de permettre de calculer en temps réel le barycentre des signaux des photomultiplicateurs avec des moyens très simples.

En effet, le système décrit précédemment comporte un nombre limité de composants. De plus, les résistances utilisées pour injecter le signal des photomultiplicateurs dans les lignes collectrices sont très peu coûteuses.

Des améliorations ponctuelles de la caméra de type ANGER ont été proposées. Le brevet US-A-5 576 547 propose une méthode pour corriger le calcul de l'énergie totale reçue par les détecteurs et en déduire une position corrigée de l'événement.

Des tables de corrections sont établies en construisant pour des positions connues d'événement des histogrammes de l'énergie captée par les différents détecteurs.

Ces tables sont ensuite utilisées pour corriger la position de l'événement.

Une caméra telle que la caméra ANGER présente cependant aussi un désavantage majeur qui est un taux de comptage réduit. On entend par taux de comptage le nombre d'événements, c'est-à-dire d'interactions entre un photon γ et le scintillateur, que la caméra est capable de traiter par unité de temps.

Une des limitations du taux de comptage provient notamment du fait que la caméra est incapable de traiter deux événements ayant lieu sensiblement simultanément en des points distincts du cristal scintillateur.

En effet, des événements simultanés mais géométriquement distincts donnent naissance à des signaux électriques qui s'empilent dans les lignes collectrices $LX^-$, $LX^+$, $LY^-$ et $LY^+$ et qui ne peuvent plus être distingués. Ces événements sont aussi "perdus" pour la formation d'une image.

Ce soucis de prise en compte et de correction des empilements est présent dans la demande de brevet européen n° 0 252 566.

Le dispositif décrit dans cette demande comporte des moyens de numérisation du signal fourni par chaque détecteur et l'énergie de chaque photodétecteur est calculée avec une correction pour tenir .compte de l'énergie ap-

**EP 0 950 197 B1**

portée par des événements se produisant alors que l'événement courant est encore en cours.

La limiatation du taux de comptage n'est pas une contrainte trop importante dans les techniques d'imagerie médicale traditionnelles. En effet, comme indiqué ci-dessus, le collimateur arrête un très grand nombre de rayons gamma et seul un petit nombre d'événements sont effectivement détectés.

Les gamma-caméras sont cependant utilisées également dans deux autres techniques d'imagerie médicale où la limitation du taux de comptage est une contrainte rédhibitoire.

Ces techniques sont les techniques dites de "correction d'atténuation par transmission" et de "PET (Position Emission Tomography) en coïncidence".

La technique de correction d'atténuation par transmission consiste à tenir compte, lors de la formation d'une image médicale, de l'atténuation propre du tissu du patient entourant l'organe examiné. Pour connaître cette atténuation, on mesure la transmission des rayonnements gamma vers une gamma-caméra à travers le corps du patient. A cet effet on fait prendre place au patient entre une source externe très active et la tête de détection de la gamma-caméra. Ainsi, lors de la mesure du rayonnement transmis, un nombre élevé d'événements ont lieu dans le cristal scintillateur. Le nombre élevé d'événements par unité de temps accroît aussi la probabilité d'avoir plusieurs événements sensiblement simultanés. Une caméra de type Anger classique se révèle alors inappropriée.

La technique de PET consiste à injecter au patient un élément tel que $F^{18}$ apte à émettre des positons. L'annihilation d'un positon et d'un électron libère deux photons $\gamma$ émis dans des directions opposées et ayant une énergie de 511 keV. Ce phénomène physique est mis à profit dans la technique d'imagerie PET. Dans cette technique on utilise une gamma-caméra avec au moins deux têtes de détection disposées de part et d'autre du patient. Les têtes de détection utilisées ne sont pas équipées de collimateur. En effet, un traitement électronique des informations, dit traitement de coïncidence, permet de sélectionner parmi les événements ceux qui coïncident temporellement, et de calculer ainsi la trajectoire des photons gamma.

Les têtes de détection sont donc soumises à des flux de rayonnement gamma élevé. Les gamma-caméras classiques de type Anger ont un taux de comptage généralement trop limité pour une telle application.

A titre indicatif, une gamma-caméra de type Anger peut fonctionner normalement avec une détection de $1.10^5$ événements par seconde, tandis qu'en imagerie PET il faut au moins $1.10^6$ événements par seconde pour un fonctionnement normal.

Une autre limitation des gamma-caméras du type Anger, décrites ci-dessus, tient au fait que le calcul du barycentre d'un événement est fixé définitivement par la construction de la tête de détection et notamment par le choix des résistances $RX^-$, $RX^+$, $RY^-$, $RY^+$ pour chaque photomultiplicateur. De même, le calcul de l'énergie est fixé par le câblage des photomultiplicateurs sur une voie commune (voie énergie).

Il est donc nécessaire de développer des dispositifs et des procédés permettant l'emploi de gamma-caméras à fort taux de comptage.

Par ailleurs, on souhaite développer des caméras pour lesquelles la détermination du barycentre, ou de la localisation, d'un événement, présente de bonnes caractéristiques de linéarité et de résolution spatiale.

Exposé de l'invention

L'invention a pour objet un procédé de détermination de la position $P_0$ d'un événement par rapport à un ensemble de N photodétecteurs, cet événement induisant un signal dans les N photodétecteurs, ce procédé comportant les étapes suivantes :

a) une étape de numérisation du signal délivré par chaque photodétecteur, et de calcul d'une valeur $N_{i,j}$ représentative de l'énergie du signal délivré par chaque photodétecteur,

b) calcul, en fonction des $N_{ij}$ et de la position des photodétecteurs, d'une position brute $P_0$ de l'événement, par rapport à l'ensemble des photodétecteurs,

c) détermination de la distance $d_{i,j}$ de chacun des photodétecteurs par rapport à la position $P_0$,

d) calcul d'une valeur corrigée $N'_{i,j}=F(N_{ij})$, où F est une fonction :

* qui réduit (respectivement : ne modifie pas) la valeur $N_{ij}$ pour le photodétecteur correspondant à $P_0$ et pour un certain nombre $N_1$ de photodétecteurs autour de $P_0$ (par exemple : les $N_1$ photodétecteurs de première couronne),
* qui accroît, ou qui ne modifie pas (respectivement : accroît) la valeur $N_{ij}$ pour un certain nombre de photodétecteurs $N_2$ situés autour des $N_1$ photodétecteurs précédents (par exemple : les $N_2$ photodétecteurs de deuxième couronne si les $N_1$ photodétecteurs. sont ceux de première couronne ; et les photodétecteurs de première couronne, ou de première et de deuxième couronnes si $N_1=0$),
* qui tend vers 0 au-delà,

**4**

e) calcul d'une nouvelle position $P_1$ de l'événement, en fonction de la position des photodétecteurs et des $Ni_j$.

Un tel procédé permet de traiter des données numérisées, et permet de produire un signal de position $P_1$ de l'événement par rapport à l'ensemble des N photodétecteurs.

Le caractère itératif du procédé selon l'invention lui confère une bonne résolution spatiale et une bonne linéarité.

Le calcul de la position brute $P_0$ de l'événement peut être réalisé en calculant les coordonnées barycentriques $(X_0, Y_0)$ dudit événement en fonction des $N_{i,j}$ et de la position $(XC_{i,j}, YC_{i,j})$ de chaque photodétecteur.

De même, le calcul de la position $P_1$ de l'événement peut être réalisé en calculant les coordonnées barycentriques $(X_1, Y_1)$ dudit événement en fonction des $N'_{i,j}$ et de la position $(XC_{i,j}, YC_{i,j})$ de chaque photodétecteur.

Le procédé selon l'invention, du fait du choix de la fonction F, réduit la contribution du photodétecteur qui se situe en face de la position présumée de l'événement (et, éventuellement, d'un certain nombre $N_1$ de photodétecteurs autour de celui-ci) : en effet, ce(s) photodétecteur(s) apporte(nt) peu d'information sur la valeur de la position, ou du barycentre de l'événement. Par ailleurs, cette fonction F accorde une prépondérance accrue aux photodétecteurs situés au-delà de celui qui correspond à la position brute $P_0$ de l'événement et des $N_1$ photodétecteurs.

On peut choisir, par exemple : $N_1=0$ ou $N_1=8$ (première couronne, notamment pour une répartition carrée des photodétecteurs).

Un traitement particulier peut être réalisé pour les photodétecteurs qui sont situés au bord du champ de N photodétecteurs. Ce traitement consiste en l'étape supplémentaire suivante : modification de la valeur de la position $(XC_{i,j}, YC_{i,j})$ en une nouvelle valeur $(XC'_{i,j}, YC'_{i,j})$, l'une au moins des valeurs $|X'|$ et $|Y'|$ étant supérieures à $|X|$ et $|Y|$. Ainsi, les photodétecteurs de bord de champ voient leur poids modifié, mais également leur position : le procédé selon l'invention modifie à la fois leur contribution et leur poids dans le calcul de la position ou du barycentre de l'événement : ceci permet un agrandissement du champ et une amélioration de la linéarité.

Les calculs de barycentre peuvent être réalisés de manière séquentielle. Le temps de traitement est alors d'autant plus long que le nombre de photodétecteurs intervenant dans le calcul est grand.

On peut, de manière alternative, réaliser un traitement parallèle des données. Ainsi, la détermination des coordonnées barycentriques $(X_0, Y_0)$ de l'événement peut comporter les sous-étapes suivantes :

$b_1$) une sous-étape de détermination, pour chaque colonne i :

- de la contribution de la colonne à l'énergie totale induite par l'événement dans l'ensemble des photodétecteurs,
- de la contribution de la colonne au barycentre, en X, de l'événement,
- de la contribution de la colonne au barycentre, en Y, de l'événement.

$b_2$) une sous-étape de détermination :

- de l'énergie totale induite par l'événement dans l'ensemble des photodétecteurs,
- des coordonnées barycentriques $(X_0, Y_0)$ de l'événement par rapport aux N photodétecteurs.

Le procédé selon l'invention permet alors l'exploitation de gamma-caméras à fort taux de comptage, ce qui est très avantageux dans le cas de mesures dites de "correction d'atténuation par transmission" ou de "PET en coïncidence". Le taux de comptage élevé est atteint sans restreindre le nombre de photodétecteurs lus. Ceci est dû au parallélisme employé, et au fort "pipelinage", c'est-à-dire à la succession d'opérations simples. Le procédé selon l'invention permet alors d'accélérer le calcul du barycentre des contributions numérisées des photodétecteurs, en parallélisant ce calcul.

Le même traitement parallèle peut être appliqué à la détermination des coordonnées barycentriques $(X_1, Y_1)$ de l'événement et avec les mêmes avantages.

Dans le cadre de l'invention, on peut évidemment remplacer les colonnes par des lignes, le principe du calcul restant le même.

Il est possible de procéder à une étape préliminaire de détection de la position présumée d'un événement. On peut alors, autour de cette position présumée, délimiter un sous-ensemble de N' photodétecteurs parmi les N photodétecteurs, seuls les signaux de ces N' photodétecteurs étant pris en compte pour réaliser les étapes b, c, d et e du procédé selon l'invention.

L'invention s'applique de manière particulièrement avantageuse dans le cas où les photodétecteurs sont des photomultiplicateurs d'une gamma-caméra. En particulier, on peut ainsi mettre en oeuvre les techniques d'imagerie en correction d'atténuation par transmission, et les techniques d'imagerie en PET en coincidence.

L'invention a également pour objet un dispositif pour mettre en oeuvre le procédé décrit ci-dessus.

Brève description des figures

De toute façon, les caractéristiques et avantages de l'invention apparaîtront mieux à la lumière de la description qui va suivre. Cette description porte sur les exemples de réalisation, donnés à titre explicatif et non limitatif, en se référant à des dessins annexés sur lesquels :

- la figure 1, déjà décrite, est une coupe schématique d'une tête de détection d'une caméra de type Anger connue ;
- la figure 2, déjà décrite, montre de façon schématique un dispositif de collection et de codage de signaux provenant de photomultiplicateurs de la tête de détection de la figure 1 ;
- la figure 3 représente l'interconnexion d'un ensemble de photodétecteurs,
- la figure 4 représente un dispositif de lecture d'un ensemble de photodétecteurs, pour la mise en oeuvre de l'invention,
- les figures 5A et 5B représentent des exemples de fonction F,
- la figure 6 représente la structure d'un système de calcul pour un mode de réalisation de l'invention,
- la figure 7 représente la structure d'un opérateur colonne pour un mode de réalisation de l'invention,
- la figure 8 représente la structure d'une partie d'un système de calcul pour un mode de réalisation de l'invention,
- la figure 9 représente une réalisation d'une autre partie d'un système de calcul pour un mode de réalisation de l'invention,
- la figure 10 représente un circuit associé à un photodétecteur, pour le traitement des données de ce photodétecteur ;
- les figures 11A et 11B représentent un signal analogique fourni par un photodétecteur (figure 10A), ainsi que le signal analogique numérisé correspondant (figure 10B) ;
- la figure 12 représente un mode de réalisation d'un dispositif pour la détermination d'une position présumée d'un événement.

Description détaillée de modes de réalisation de l'invention

L'invention va être décrite de manière détaillée pour des photomultiplicateurs d'une gamma-caméra. Cependant, cette description vaut aussi pour des photodétecteurs quelconques, ne faisant pas nécessairement partie d'une gamma-caméra.

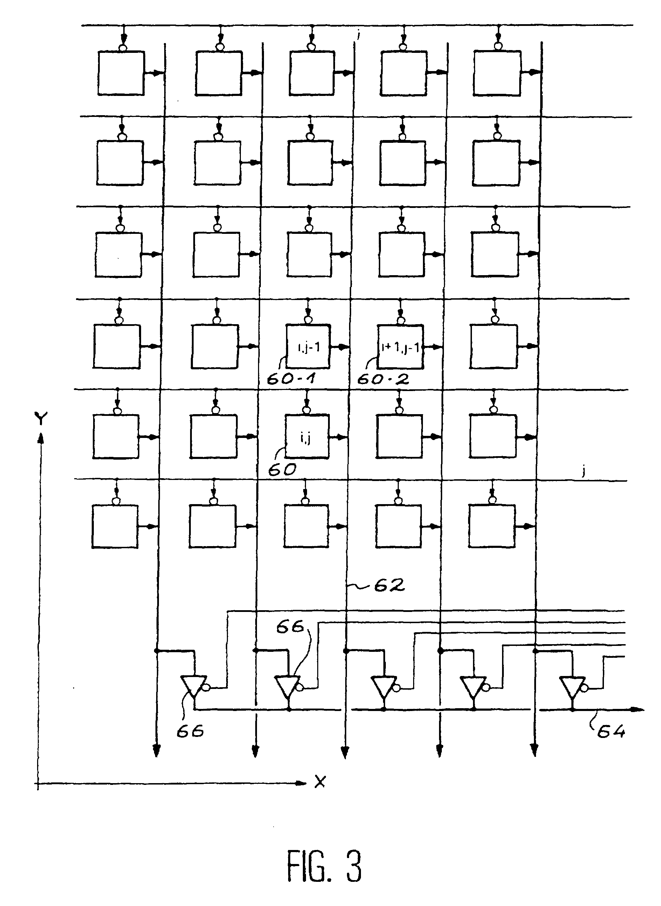

La figure 3 représente un ensemble de photomultiplicateurs 60, 60-1, 60-2,... constituant une tête de gamma-caméra. Chaque photomultiplicateur est repéré par sa position (i,j) dans l'ensemble des photomultiplicateurs. Plus précisément, on désigne par $XC_{i,j}$ et $YC_{i,j}$ les coordonnées, suivant deux axes X, Y, du centre du photomultiplicateur i, j.

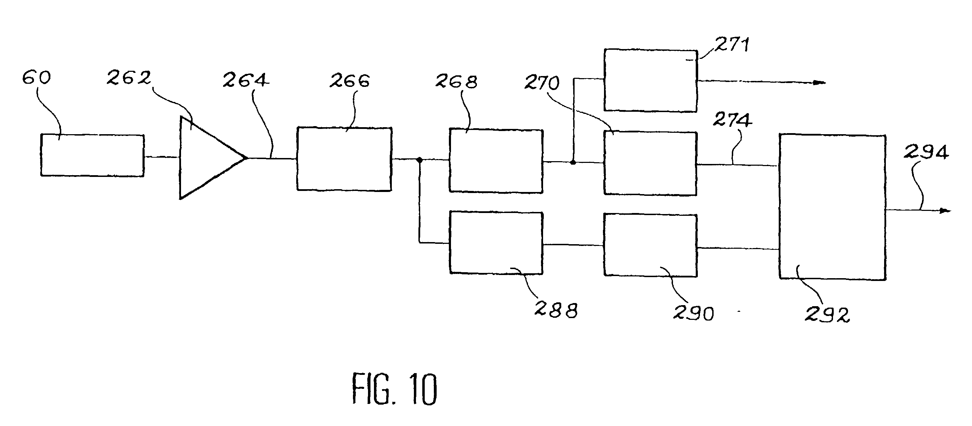

Le signal issu de chacun des photomultiplicateurs est numérisé et traité individuellement (intégration, corrections, etc.). Chaque photomultiplicateur possède un niveau de registre de stockage qui permet de mémoriser la contribution de chaque photomultiplicateur lors de la détection d'un événement. Ces aspects seront traités plus loin en liaison avec les figures 10-11B.

Le réseau de photomultiplicateurs est organisé en lignes et en colonnes, et toutes les sorties des registres de stockage des photomultiplicateurs d'une même colonne sont connectés sur un bus 62 (bus colonne). Les bus colonne peuvent être rassemblés en un bus série 64.

Un opérateur réalisant les diverses étapes du procédé selon l'invention peut être connecté sur le bus série. Cet opérateur travaille alors en série, le temps de traitement étant d'autant plus long que le nombre de photodétecteurs intervenant dans le calcul est grand.

Il peut être également prévu de réaliser un traitement en parallèle : un opérateur réalisant les diverses étapes du procédé selon l'invention est connecté sur les bus colonne 62. On obtient ainsi un meilleur taux de comptage.

Lorsque le réseau de photomultiplicateurs n'est pas trop important, on peut prendre en compte l'ensemble des photomultiplicateurs.

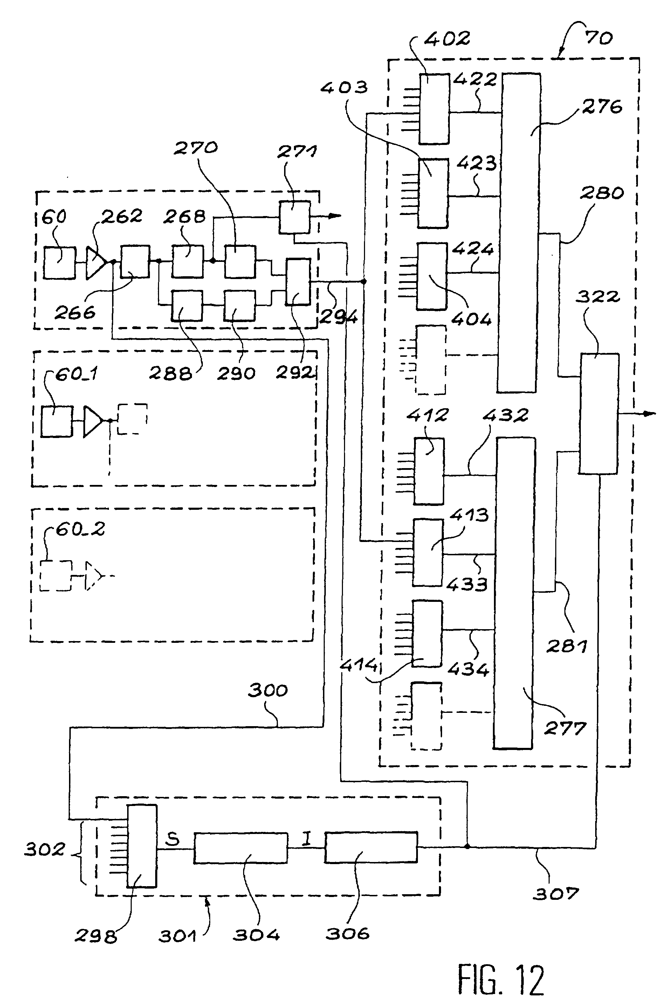

Lorsque le réseau de photomultiplicateurs est important (par exemple entre 50 et 100 photomultiplicateurs), une position grossière de l'événement est d'abord déterminée, par exemple par une méthode qui sera décrite plus loin, en liaison avec la figure 12.

Le fait de connaître la zone d'interaction de l'événement permet déjà de limiter le nombre de photomultiplicateurs à prendre en compte pour calculer la position de l'interaction. En effet, seules les deux couronnes de photomultiplicateurs qui entourent le photomultiplicateur lieu de l'interaction portent en général de l'information significative.

La précision de la position présumée étant insuffisante, on est cependant souvent appelé à lire une zone plus large que celle strictement nécessaire (par exemple souvent jusqu'à 25 ou 30 lectures par événement). La durée de lecture des registres de stockage pour traiter un événement est un passage obligé qui influe directement sur le taux de comptage de la machine (nombre d'événements traités par seconde).

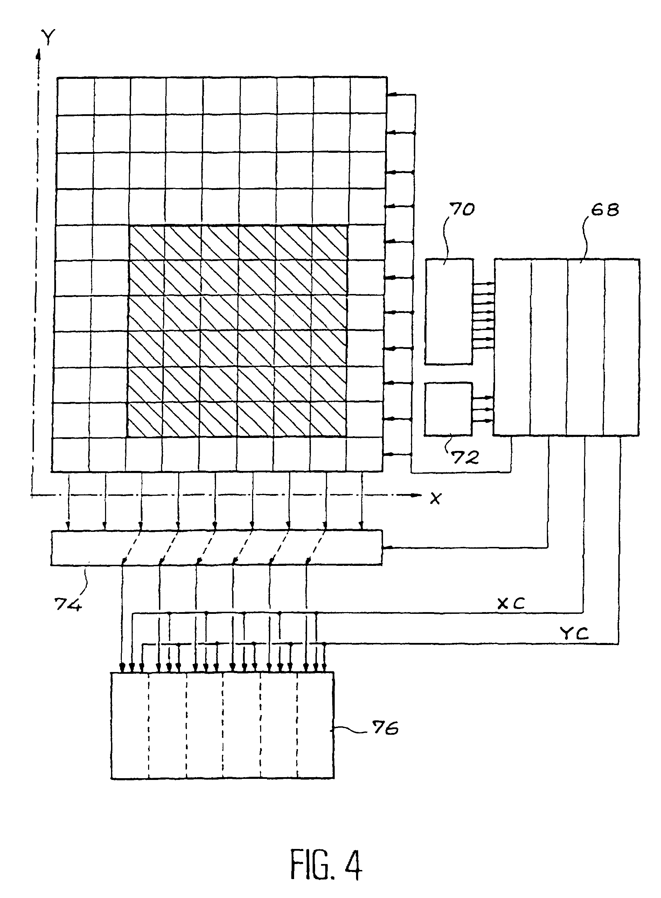

Dans le cas du traitement en parallèle, des moyens 66 permettent de sélectionner chaque colonne indépendamment des autres. Ces moyens 66 sont par exemple commandés par un séquenceur de lecture 68 (figure 4).

La figure 4 illustre de manière plus précise un dispositif permettant de mettre en oeuvre le traitement en parallèle.

Un séquenceur de lecture 68 permet de lire le contenu du registre de stockage de chaque photomultiplicateur. Soit $N_{i,j}$ le contenu du registre de stockage du photomultiplicateur de colonne i et de ligne j. $N_{i,j}$ représente en fait, par exemple, une intégrale numérique du signal délivré par le photomultiplicateur i,j en réponse à un événement.

Des moyens 70 permettent de déterminer une position présumée, ou grossière de l'événement. Ces moyens seront décrits plus loin de manière plus détaillée (figure 12).

Le séquenceur de lecture 68 commande l'adressage des colonnes par un multiplexeur 74. La contribution de chaque colonne à l'énergie totale, à la composante en X du barycentre (XC) et à la composante en Y du barycentre (YC) est transférée à un système de calcul 76, soit par l'adressage des colonnes via le multiplexeur, soit directement par le séquenceur de calcul 68.

Le nombre de colonnes et de lignes pris en compte autour de chaque position présumée est imposé par l'imprécision de la détermination. Sur la figure 4, ce sont 36 (6x6) photomultiplicateurs qui sont pris en compte. D'une manière générale, pour un champ de N photomultiplicateurs on peut être amené à prendre en compte $N_1$ photomultiplicateurs ($N_1 < N$) en fonction de la précision voulue : sur la figure 4, N=11x9 et $N_1$=6x6.

Par conséquent, à chaque événement correspond une position présumée (PP) et, à chaque position présumée, on fait correspondre un ensemble de $N_1$ (36) photomultiplicateurs situés sur 6 lignes et 6 colonnes successives de façon à ce que tous les photomultiplicateurs qui ont stocké de l'information soient retenus. A chaque pas de lecture (tous les 100 nsec), le séquenceur de lecture 68 fournit, en fonction de la position présumée :

- les commandes nécessaires au multiplexeur 74 pour qu'il puisse orienter les bus colonnes à lire vers l'organe de calcul 76,
- les signaux de sélection de ligne de façon à présenter sur les bus colonne les registres de stockage commandés par la ligne n pour le premier temps de lecture, puis ceux commandés par la ligne n+1 pour le deuxième, et ceci jusqu'à ceux de la ligne n+5 pour le sixième temps de lecture,
- les coordonnées XC et YC des centres des photomultiplicateurs présentés sur les bus colonne.

Le séquenceur peut être réalisé sous forme d'EPROM. A chaque position présumée correspond une page mémoire dans laquelle on décrit les commandes et les valeurs nécessaires au calcul. Cette page est lue ligne par ligne à l'aide d'un compteur 72 qui active les adresses basses des EPROM.

Le calcul de l'énergie et/ou de la position brute $P_0$, en X et en Y, d'un événement peut être résumé de la manière suivante :

α) l'énergie de l'événement est la somme des contributions de tous les photomultiplicateurs qui entourent la position présumée :

$$E = \sum_{i,j} N_{i,j}$$

Cette expression peut aussi s'écrire :

$$E = \sum_{i} \left[ \sum_{j} N_{i,j} \right], \quad \text{où} \quad \sum_{j} N_{i,j}$$

représente la somme des contributions des 6 photomultiplicateurs de la colonne i, l'énergie E étant la somme des énergies obtenues sur les 6 colonnes.

β) La position $P_0$ de l'événement est calculée par la méthode du barycentre :

•

$$X_0 = \sum_{i,j} \left( XC_{i,j} * N_{i,j} \right) / \sum_{i,j} N_{i,j},$$

où $XC_{i,j}$ est la coordonnée en X du centre du photomultiplicateur situé sur la colonne i et la ligne j,

$$Y_0 = \sum_{i,j} \left( YC_{i,j} * N_{i,j} \right) / \sum_{i,j} N_{i,j},$$

où $YC_{i,j}$ est la coordonnée en Y du centre du photomultiplicateur situé sur la colonne i et la ligne j,
Comme précédemment, on peut aussi écrire :

$$X_0 = \sum_{i} \left[ \sum_{j} \left( XC_{i,j} * N_{i,j} \right) \right] / \sum_{i,j} N_{i,j}, \quad \text{où} \quad \sum_{j} \left( XC_{i,j} * N_{i,j} \right)$$

est la contribution au barycentre en X des 6 photomultiplicateurs de la colonne i,

$$Y_0 = \sum_{i} \left[ \sum_{j} \left( YC_{i,j} * N_{i,j} \right) \right] / \sum_{i,j} N_{i,j}, \quad \text{où} \quad \sum_{j} \left( YC_{i,j} * N_{i,j} \right)$$

est la contribution au barycentre en Y des 6 photomultiplicateurs de la colonne i.

On appelle barycentre brut $P_0$ le résultat obtenu à l'aide des étapes $\alpha$) et $\beta$) décrites ci-dessus, par opposition au barycentre pondéré.

Une fois le barycentre brut $P_0(X_0, Y_0)$ connu, on peut calculer la distance $d_{i,j}$ du centre de chaque photomultiplicateur à $P_0$ et pondérer la valeur $N_{i,j}$ en calculant un nouvel $N_{i,j}$ appelé $N'_{i,j}$, tel que $N'_{i,j} = F(N_{i,j})$, où F est une fonction de $d_{i,j}$ (fonction de pondération).

Cette fonction F est déterminée de manière empirique et adaptée à chaque type de photomultiplicateur et à chaque géométrie de collection de la lumière.

La fonction F, généralement:

- est inférieure à 1, pour $d_{i,j}$ faible (pour minimiser la contribution du photomultiplicateur qui reçoit l'événement quand celui-ci est proche de son centre, ce photomultiplicateur contribuant peu à l'information sur le barycentre),
- est supérieure à 1 quand $d_{i,j}$ est de l'ordre de grandeur de la dimension du photomultiplicateur (pour renforcer la contribution des photomultiplicateurs de première couronne, c'est-à-dire des photomultiplicateurs les plus proches du photomultiplicateur qui reçoit l'événement),
- tend vers zéro, lorsque $d_{i,j}$ devient grand (pour diminuer la contribution des photomultiplicateurs au fur et à mesure qu'ils sont plus loin du lieu de l'interaction, car le rapport signal/bruit de leur contribution devient de plus en plus mauvais).

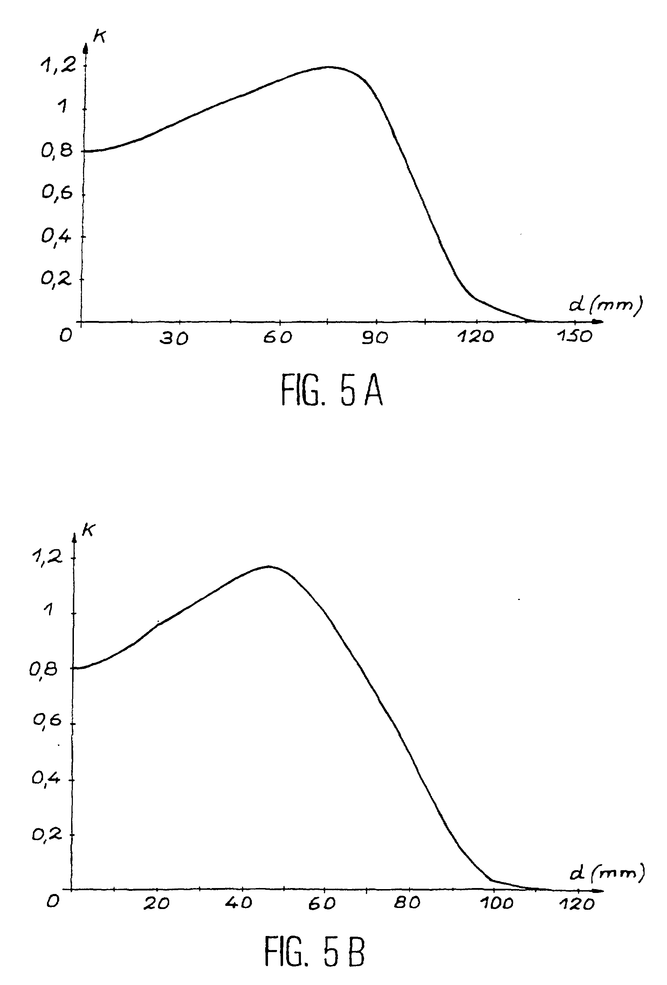

Un premier exemple de fonction F(d) est donné sur la figure 5A. Ce premier exemple correspond à des photomultiplicateurs carrés de 75 mm. Des valeurs de F, pour des valeurs particulières de d (avec un pas de 5 mm), sont données dans le tableau I ci-dessous.

TABLEAU I

| d | F | d | F |
|---|---|---|---|
| 0 | 0,8 | 80 | 1,18 |
| 5 | 0,809 | 85 | 1,13 |
| 10 | 0,826 | 90 | 1 |
| 15 | 0,85 | 95 | 0,831 |
| 20 | 0,883 | 100 | 0,663 |
| 25 | 0,916 | 105 | 0,494 |

TABLEAU I   (suite)

| d | F | d | F |
|---|---|---|---|
| 30 | 0,95 | 110 | 0,325 |
| 35 | 0,983 | 115 | 0,117 |
| 40 | 1,016 | 120 | 0,1 |
| 45 | 1,05 | 125 | 0,065 |
| 50 | 1,083 | 130 | 0,035 |
| 55 | 1,116 | 135 | 0,015 |
| 60 | 1,15 | 140 | 0 |
| 65 | 1,176 | 145 | 0 |
| 70 | 1,194 | 150 | 0 |
| 75 | 1,2 | - | - |

Un second exemple de fonction F(d) est donné sur la figure 5B. Ce second exemple correspond à des photomultiplicateurs hexagonaux de 60 mm. Des valeurs de F, pour des valeurs particulières de d (avec un pas de 5 mm), sont données dans le tableau II ci-dessous.

TABLEAU II

| d | F | d | F |
|---|---|---|---|
| 0 | 0,8 | 65 | 0,863 |
| 5 | 0,813 | 70 | 0,744 |
| 10 | 0,856 | 75 | 0,619 |
| 15 | 0,906 | 80 | 0,475 |
| 20 | 0,963 | 85 | 0,319 |
| 25 | 1,006 | 90 | 0,181 |
| 30 | 1,056 | 95 | 0,081 |
| 35 | 1,106 | 100 | 0,025 |
| 40 | 1,15 | 105 | 0,013 |
| 45 | 1,169 | 110 | 0 |
| 50 | 1,144 | 115 | 0 |
| 55 | 1,075 | 120 | 0 |
| 60 | 0,975 | - | - |

Du point de vue de la réalisation, le calcul du barycentre pondéré $P_1$ est réalisé de la même manière que le barycentre brut $P_0$, après avoir remplacé $N_{i,j}$ par $N'_{i,j}$. On fait donc suivre le calcul du barycentre brut par une opération de pondération avec la fonction F.

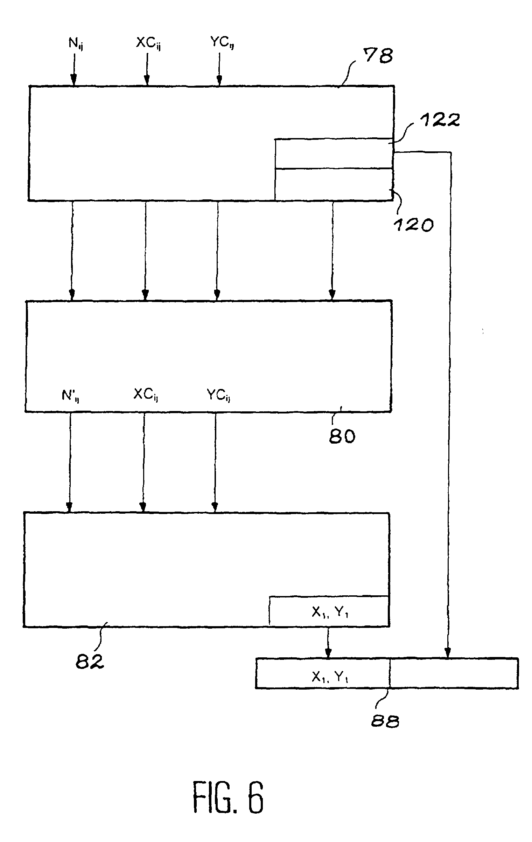

Un dispositif ou système de calcul 76 pour la mise en oeuvre du procédé selon l'invention peut comporter trois ensembles représentés schématiquement sur la figure 6 et désignés respectivement par les références 78, 80 et 82.

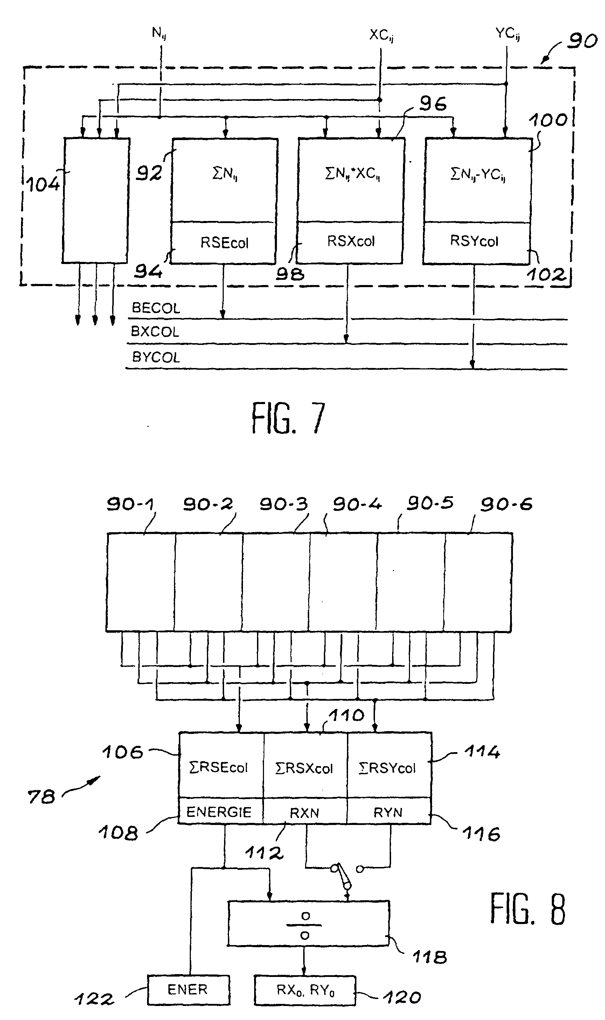

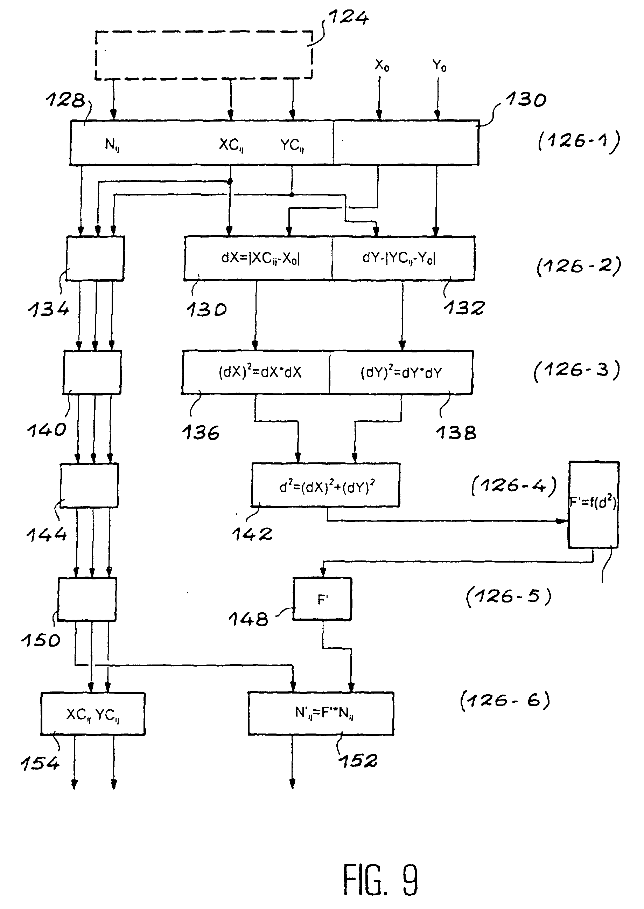

La structure du dispositif 78 permettant de déterminer $P_0$ va être décrite de manière plus précise en liaison avec les figures 7 et 8.

La figure 7 représente des moyens 90 associés à chaque colonne et dénommés par la suite opérateur colonne.

Celui-ci reçoit, en plus des valeurs $N_{i,j}$ des photomultiplicateurs de la colonne, les coordonnées $XC_{i,j}$ et $YC_{i,j}$ des centres des photomultiplicateurs correspondants, par exemple fournies par le séquenceur de lecture 68. Les coordonnées en X ne sont pas obligatoirement identiques pour une même colonne car elles doivent tenir compte de la position réelle du photomultiplicateur au sein du champ de la gamma-caméra. Il en va de même pour les coordonnées en Y. Dans l'exemple donné, chaque sortie de bus colonne est connecté à l'entrée d'un opérateur colonne 90.

Chaque opérateur colonne 90 accomplit trois opérations, de préférence en parallèle.

Une première opération consiste à calculer la contribution de la colonne à l'énergie. Par exemple, après avoir été initialisé en début de séquence, un accumulateur 92 fait la somme des valeurs $N_{i,j}$ des 6 photomultiplicateurs de la colonne et stocke le résultat dans un registre 94 (RSEcol). Les sorties des 6 registres (RSEcol1 à RSEcol6) sont regroupées sur un bus commun BECOL.

Une deuxième opération consiste à calculer la contribution de la colonne au barycentre en X. Par exemple, après avoir été initialisé en début de séquence, un multiplieur-accumulateur 96 effectue la somme des contributions au barycentre en X des 6 photomultiplicateurs de la colonne, et stocke le résultat dans un registre 98 (RSXcol). Les sorties des 6 registres (RSXcol1 à RSXcol6) sont regroupées sur un bus commun BXCOL.

Une troisième opération consiste à calculer la contribution de la colonne au barycentre en Y. Par exemple, après avoir été initialisé, un second multiplieur-accumulateur 100 effectue la somme des contributions au barycentre en Y des 6 photomultiplicateurs de la colonne, et stocke le résultat dans un registre 102 (RSYcol). Les sorties des 6 registres (RSYcol1 à RSYcol6) sont regroupées sur un bus commun BYCOL.

A la fin des 6 temps de lecture, les 6 opérateurs colonne ayant effectué leur travail deviennent disponibles pour une nouvelle lecture puisque les résultats de la première lecture sont mémorisés.

En parallèle à ces opérations de calcul, les valeurs $N_{i,j}$ des photomultiplicateurs ainsi que les coordonnées $XC_{i,j}$ et $YC_{i,j}$ peuvent être mémorisées dans un système 104 de type FIFO de façon à pouvoir être utilisées par la suite.

Un regroupement des contributions à l'énergie et aux coordonnées du barycentre en X et en Y est ensuite effectué. Ce regroupement va être décrit à l'aide de la figure 8 où les références 90-1, ..., 90-6 désignent 6 opérateurs colonne du type décrit ci-dessus en liaison avec la figure 7.

Un accumulateur 106, après avoir été initialisé en début de séquence, alimenté par le bus BECOL, effectue la somme des six registres RSEcol1 à RSEcol6 et stocke le résultat dans un registre 108 (ENERGIE). Le contenu de ce registre représente la somme des contributions à l'énergie des 36 photomultiplicateurs entourant la position présumée, donc l'énergie de l'événement.

Un second accumulateur 110, alimenté par le bus BXCOL, effectue la somme des six registres RSXcol1 à RSXcol6, et stocke le résultat dans un registre 112 (RXN). Le contenu de ce registre représente

$$\sum_i \left[ \sum_j (XC_{ij} * N_{ij}) \right].$$

Un troisième accumulateur 114, alimenté par le bus BYCOL, effectue la somme des six registres RSYcol1 à RSYcol6, et stocke le résultat dans un registre 116 (RYN). Le contenu de ce registre représente

$$\sum_i \left[ \sum_j (YC_{ij} * N_{ij}) \right].$$

Les registres RSEcol, RSXcol et RSYcol sont ensuite libérés de façon à pouvoir être utilisés par les opérateurs colonne, et les accumulateurs sont ainsi de nouveau disponibles pour traiter un nouvel événement.

Enfin, les coordonnées $X_0$ et $Y_0$ du point d'interaction $P_0$ de l'événement sont calculées en effectuant deux divisions à l'aide d'un diviseur 118 :

$$X_0 = RXN/ENERGIE,$$

et

$$Y_0 = RYN/ENERGIE,$$

et ceci en moins de 6 temps de lecture pour pouvoir libérer les registres de stockage 108, 112, 116. Les diviseurs intégrés pipelinés du commerce (de type RAYTHEON 3211 par exemple) sont capables d'assumer largement ces performances et permettent même de n'utiliser qu'un seul boîtier, en effectuant les deux divisions successivement.

On obtient donc la position $P_0$ de l'événement dont les coordonnées $X_0$ et $Y_0$ sont stockées dans un registre 120 ($RX_0$ et $RY_0$). Parallèlement aux divisions, l'énergie peut être pipelinée depuis le registre 108 vers un registre 122 de façon à libérer le registre 108 pour l'événement suivant.

Une réalisation simple du dispositif 80 (calcul du barycentre pondéré : figure 6) est donnée en figure 9.

Les valeurs de $N_{i,j}$, $XC_{i,j}$, $YC_{i,j}$, qui intervenaient dans le calcul du barycentre brut ont été mémorisées dans des mémoires FIFO désignées globalement par la référence 124 (on a déjà mentionné ci-dessus la mémoire 104 (figure 7) dans laquelle $N_{i,j}$ est stockée). Le calcul est organisé selon les étapes 126-1, ..., 126-6 suivantes, pour chaque opérateur pondération travaillant sur une colonne :

- 126-1 : récupération de la première valeur $N_{i,j}$ et des coordonnées du centre du photomultiplicateur correspondant $XC_{i,j}$ et $YC_{i,j}$ (première valeur écrite dans la FIFO, donc première valeur lue) et stockage dans un registre d'entrée 128 de l'opérateur de pondération. Parallèlement mise en mémoire 130, à l'entrée de l'opérateur, de $X_0$ et $Y_0$. Cette mémorisation n'est pas répétée pour les 5 acquisitions de $N_{i,j}$, $XC_{i,j}$ et $YC_{i,j}$ suivantes puisque $P_0$ reste le même.
- 126-2 : calcul de $dX=|XC_{i,j}-X_0|$, et parallèlement de $dY=|YC_{i,j}-Y_0|$, et mise en mémoire 130, 132, 134, de dX, dY, $N_{i,j}$, $XC_{i,j}$ et $YC_{i,j}$. Il y a ensuite libération des registres servant à l'étape 126-1, qui peuvent alors recueillir les valeurs relatives au photomultiplicateur suivant. Celui-ci est traité comme le précédent et ainsi de suite jusqu'au sixième.
- 126-3 : calcul de $(dX)^2=dX*dX$, et parallèlement de $(dY)^2=DY*DY$, et mise en mémoire 136, 138, 140 de $(dX)^2$, $(dY)^2$, $N_{i,j}$, $XC_{i,j}$, et $YC_{i,j}$. Puis il y a libération des registres de sortie de l'étape 126-2,
- 126-4 : calcul de $d^2=(dX)^2+(dY)^2$ et stockage de $d^2$, $N_{i,j}$, $XC_{i,j}$ et $YC_{i,j}$ dans des registres 142, 144 ; puis, libération des registres de sortie de l'étape 126-3.
- 126-5 : adressage d'une EPROM 146 contenant la fonction $F'=f'(d^2)$, par le registre contenant $d^2$. Ceci évite d'avoir à extraire la racine carrée de $d^2$, sachant qu'il est facile d'obtenir $F'=f'(d^2)$ quand on connaît $F=f(d)$. Il y a ensuite stockage de F', $N_{i,j}$, $XC_{i,j}$ et $YC_{i,j}$ dans . des registres 148, 150 et libération des registres de sortie de l'étape 126-4.
- 126-6 : calcul de $N'_{i,j}=F'*N_{i,j}$ et stockage de $N'_{i,j}$, $XC_{i,j}$ et $YC_{i,j}$ dans des registres 152, 154 ; puis il y a libération des registres de sortie de l'étape 126-5.

Ainsi découpé et pipeliné le calcul de $N'_{i,j}$ est facilement réalisable car chaque pas de calcul est suffisamment simple pour être réalisé pendant la durée d'un pas de lecture (typiquement 100 nsec).

Le troisième sous-système de calcul 82 (figure 6), pour calculer le barycentre pondéré $X_1$, $Y_1$, a une architecture du type de celle décrite ci-dessus en liaison avec les figures 7 et 8.

La sortie du barycentre pondéré donne les nouvelles coordonnées $X_1$, $Y_1$ de la position de l'événement. On peut faire progresser la valeur de l'énergie parallèlement aux calculs ; on obtient en sortie finale, dans un même registre 88, l'énergie et les coordonnées de l'événement.

Il reste à décrire comment le signal issu de chaque photomultiplicateur est détecté et traité, en en particulier comment une position présumée de l'événement peut être calculée.

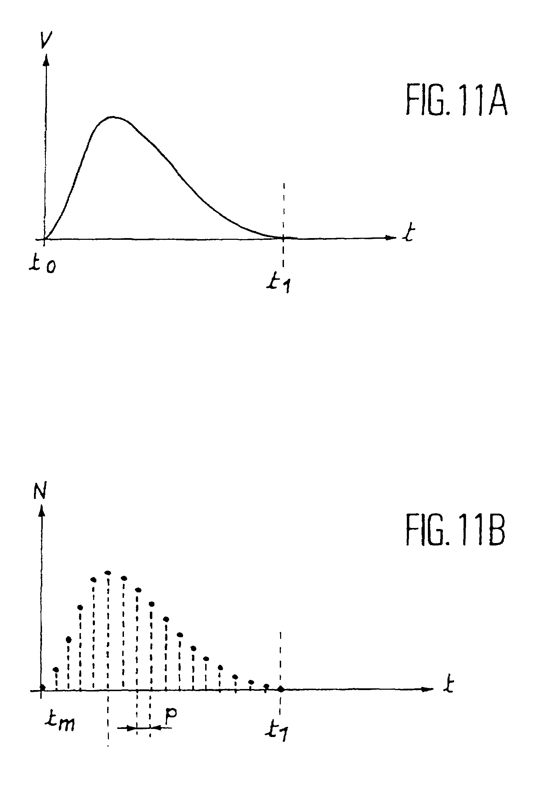

La figure 10 représente la partie du dispositif associée à un unique photomultiplicateur 60. Le photomultiplicateur 60 relié à un convertisseur courant-tension 262. En réponse à un événement détecté par le photomultiplicateur, on obtient un signal sur la sortie 264 du convertisseur courant-tension 262, par exemple du type de celui qui est illustré sur la figure 11A.

Le graphique de la figure 11A indique, en ordonnées, l'amplitude du signal correspondant à l'impulsion et, en abscisses, le temps. L'amplitude du signal et le temps sont indiqués en échelle arbitraire. $t_0$ désigne l'instant de départ de l'impulsion fournie par le photodétecteur et $t_1$ l'instant où l'impulsion redevient quasiment nulle, après être passée par un maximum. A titre indicatif, la durée correspondant à l'intervalle $t_1 - t_0$ est de l'ordre d'une microseconde, dans le cas d'un photomultiplicateur d'une gamma-caméra couplé à un cristal de NaI (T$\ell$).

Le signal analogique présent sur la borne de sortie 264 est dirigé vers un convertisseur analogique-numérique 266. Ce dernier échantillonne chaque impulsion du signal en un certain nombre d'échantillons n, comme illustré sur la figure 10B. Deux échantillons consécutifs sont séparés par un pas, ou intervalle d'horloge p (l'horloge fonctionnant à 1/p Hz).

A titre d'exemple, le convertisseur échantillonne chaque impulsion du signal en n = 10 échantillons. Pour un signal de 1 microseconde, un échantillonnage est alors effectué toutes les 100 nanosecondes.

Le convertisseur analogique-numérique 266 est, de préférence, un convertisseur rapide, de type « flash » pouvant fonctionner à une fréquence de l'ordre de 10 à 20 mégahertz.

Le signal numérique issu du convertisseur analogique-numérique 266 dirigé vers un sommateur numérique 268. Ce sommateur effectue une somme glissante des échantillons qui lui sont transmis par le convertisseur analogique-numérique 266. La somme glissante est effectuée sur un nombre donné d'échantillons. Ce nombre prédéterminé est égal, par exemple, à 10.

Pour chaque photodétecteur i,j, cette somme glissante, ou l'intégrale numérique du signal associé à l'événement correspond à la grandeur $N_{i,j}$ déjà introduite ci-dessus.

Parallèlement, le résultat de la sommation effectuée avec les moyens 268 est stocké dans un registre 271. La fonction de stockage peut être composée de plusieurs registres pour permettre de mémoriser plusieurs événements temporellement très proches.

La valeur de la somme glissante est dirigée vers des moyens de comparaison 270. La valeur de la somme glissante y est comparée avec une valeur seuil prédéterminée fixée à une entrée 272 du comparateur 270. Ce comparateur émet sur une sortie 274 un signal binaire, représentatif du résultat de la comparaison (par exemple, 0 si la valeur de la somme glissante est inférieure à la valeur de référence fixée et 1 si la valeur de la somme glissante est supérieure à la valeur de référence).

De façon à limiter la durée de ce dépassement, celui-ci ne sera validé que pendant une fenêtre temporelle centrée sur le maximum de la somme glissante. Ceci permet de séparer des événements proches dans le temps mais géographiquement distincts sur le champ du détecteur.

Cette fenêtre est positionnée en prenant comme référence l'instant de passage du signal codé par un maximum. Cette détection est réalisée par les moyens 288 en comparant la valeur courante de la sortie du codeur à la valeur précédente. Lorsque la valeur courante est inférieure à la valeur précédente, le comparateur 288 émet une impulsion. Cette impulsion est envoyée à un registre à décalage 290 dont on règle le retard $n_1$ pour générer une fenêtre temporelle centrée sur le maximum de la somme glissante. Pour tenir compte de l'imprécision (plus ou moins un pas d'échantillonnage) de la détermination de la position du maximum du signal codé, la fenêtre temporelle sera activée pendant $n_0$ pas d'échantillonnage avec $n_0 \geq 3$ (par exemple $n_0=3$), ce choix d'un minimum de trois garantissant un minimum de simultanéité des signaux de dépassement de seuil entre les photomultiplicateurs activés par un même événement.

Une porte ET 292, dont les entrées sont le signal obtenu en sortie du comparateur 270, et le signal de sortie du registre à décalage 290, permet d'obtenir, sur sa sortie 294, un signal de dépassement de seuil à l'instant voulu par rapport au passage par le maximum du signal numérique.

La figure 12 représente un dispositif, conforme à l'invention, pour le traitement des signaux issus de plusieurs photodétecteurs 60, 60-1, 60-2. Sur cette figure, des références identiques à celles de la figure 10 y désignent des éléments similaires ou correspondants.

Sur cette figure, on voit qu'il est possible de prélever, en sortie du convertisseur courant-tension 262, un signal analogique 300, du type de celui qui est décrit ci-dessus en liaison avec la figure 11A. Sur la figure 12, la référence 302 désigne globalement l'ensemble des signaux analogiques prélevés sur les autres convertisseurs courant-tension 262-1, 262-2, .... L'ensemble de ces signaux rentrent dans un sommateur analogique 298 qui délivre un signal S, somme de tous les signaux analogiques fournis par un certain nombre de photodétecteurs, par exemple par tous les photodétecteurs. Un dispositif 304 permet de délivrer une impulsion I lors du passage du signal S par son maximum. Ce dispositif 304 comporte, par exemple, un différentiateur (capacité, amplificateur et résistances entre l'entrée et la sortie de l'amplificateur) ; la sortie de ce différentiateur alimente un comparateur qui permet de détecter le passage à 0 de la sortie du différentiateur. L'impulsion I alimente l'entrée d'un registre à décalage 306 dont le pas p est réglé par l'horloge H. La sortie 307 de ce registre est dénommée impulsion de mémorisation et permet, en particulier, de déclencher le registre de mémorisation 271 correspondant au photodétecteur 60. Il déclenche également chaque registre de mémorisation associé à cnaque photodétecteur. Le retard du registre à décalage 306 est réglé de façon à ce que le front montant du signal de mémorisation 307 soit synchrone de l'instant où l'on doit mémoriser les sommes dans les registres 271.

L'ensemble des photodétecteurs 60, 60-1, 60-2, ... est réparti par exemple en un réseau bidimensionnel.

Afin de repérer la position présumée (ou grossière) d'un événement par rapport à ce réseau bidimensionnel de photodétecteurs, on associe avantageusement une mémoire à accès lecture à une première direction de repérage dans le réseau de photodétecteurs et une mémoire à accès lecture à une seconde direction de repérage dans le réseau des photodétecteurs. Si on repère ce réseau par des lignes et des colonnes, on peut donc ainsi associer une mémoire à accès lecture pour repérer une coordonnée « ligne » et une mémoire à accès lecture pour repérer une coordonnée « colonne ».

Plus précisément, dans un dispositif selon l'invention, du type ce celui illustré en figure 12, les sorties 294 qui représentent, lorsqu'elles sont actives, les photodétecteurs du centre de l'interaction, sont utilisées de la manière suivante :

- un circuit OU (402) regroupe les sorties de type 294 des photodétecteurs d'une même colonne et génère un signal 422 actif lorsqu'au moins une de ses entrées est active. Il y a autant de circuits de type 402 que de colonnes,
- un circuit OU (412) regroupe les sorties de type 294 des photodétecteurs d'une même ligne et génère un signal 432 actif lorsqu'au moins une de ses entrées est active. Il y a autant de circuits de type 412 que de lignes.

Les signaux de type 422 sont les adresses d'une PROM 276 qui est programmée de façon à fournir la coordonnée (280) de la position présumée par rapport aux colonnes. De même, les signaux de type 432 sont les adresses d'une deuxième PROM 277 qui est programmée de façon à fournir la coordonnée (281) de la position présumée par rapport aux lignes. La position présumée, représentée par le couple de valeurs (280, 281), est mémorisée dans un registre 322, en même temps que l'on mémorise la contribution de tous les photodétecteurs dans leurs registres respectifs (271). Cette mémorisation est déclenchée par le signal 307 généré par le registre 306.

## Revendications

1. Procédé de détermination de la position $P_1$ d'un événement dans un système de coordonnée X, Y au moyen d'un ensemble de N photodétecteurs disposés en lignes et colonnes, un quelconque détecteur d'une colonne de rang i et d'une ligne de rang j, étant repérable dans le système de coordonnée X, Y, l'événement induisant un signal dans les N photodétecteurs, le procédé comportant les étapes suivantes :

   a) numérisation du signal délivré par chaque photodétecteur, et calcul d'une valeur $N_{i,j}$ représentative de l'énergie délivrée par chaque photodétecteur,
   b) calcul, en fonction des $N_{i,j}$ et de la position des photodétecteurs, d'une position brute $P_0$ ($X_0$, $Y_0$) de l'événement, par rapport à l'ensemble des photodétecteurs,
   c) détermination de la distance $d_{i,j}$ de chacun des photodétecteurs par rapport à la position $P_0$, le procédé étant **caractérisé en ce qu'**il comporte en outre,
   d) le calcul d'une valeur corrigée $N'_{i,j}=F'(N_{i,j})$, où F' est une fonction :

   * qui réduit (respectivement : ne modifie pas) la valeur $N_{i,j}$ pour le photodétecteur correspondant à $P_0$ et pour un certain nombre $N_1$ de photodétecteurs autour de $P_0$,
   * qui accroît, ou qui ne modifie pas (respectivement : accroît) la valeur $N_{i,j}$ pour un certain nombre de photodétecteurs $N_2$ situés autour des $N_1$ photodétecteurs précédents,
   * qui tend vers 0 au-delà,

   e) calcul de la position $P_1$ de l'événement, en fonction de la position des photodétecteurs et des $N'_{i,j}$.

2. Procédé selon la revendication 1, où le calcul de la position brute $P_0$ de l'événement est réalisé en calculant les coordonnées barycentriques ($X_0$, $Y_0$) dudit événement en fonction de $N_{i,j}$ et de la position ($XC_{i,j}$, $YC_{i,j}$) de chaque photodétecteur.

3. Procédé selon la revendication 1 ou 2, où le calcul de la position $P_1$, de l'événement est réalisé en calculant les coordonnées barycentriques ($X_1$, $Y_1$) dudit événement en fonction des $N'_{i,j}$ et de la position ($XC_{i,j}$, $YC_{i,j}$) de chaque photodétecteur.

4. Procédé selon l'une des revendications 1 à 3, comportant, avant l'étape e), et pour les photodétecteurs situés au bord du champ de N photodétecteurs, une étape :

   $e_0$) de modification de la valeur de la position ($XC_{i,j}$, $YC_{i,j}$) le nouveau couple de valeurs ($XC'_{i,j}$, $YC'_{i,j}$) étant tel qu'au moins l'une des deux inégalités suivantes est satisfaite : $|XC'_{i,j}|>|XC_{i,j}|$, $|YC'_{i,j}|>|YC_{i,j}|$.

5. Procédé selon l'une des revendications 2 ou 3, les calculs de barycentre étant réalisés de manière séquentielle.

6. Procédé selon la revendication 2, la détermination des coordonnées barycentriques ($X_0$, $Y_0$) de l'événement comportant les sous-étapes suivantes :

   $b_1$) une sous-étape de détermination, pour chaque colonne i :

   - de la contribution de la colonne à l'énergie totale induite par l'événement dans l'ensemble des photodétecteurs,
   - de la contribution de la colonne au barycentre, en X, de l'événement,
   - de la contribution de la colonne au barycentre, en Y, de l'événement.

   $b_2$) une sous-étape de détermination :

- de l'énergie totale induite par l'événement dans l'ensemble des photodétecteurs,
- des coordonnées barycentriques $(X_0, Y_0)$ de l'événement par rapport aux N photodétecteurs.

7. Procédé selon la revendication 3, la détermination des coordonnées barycentriques $(X_1, Y_1)$ de l'événement comporte les sous-étapes suivantes :

$e_1$) une sous-étape de détermination, pour chaque colonne i :

- de la contribution de la colonne à l'énergie totale induite par l'événement dans l'ensemble des photodétecteurs,
- de la contribution de la colonne au barycentre, en X, de l'événement,
- de la contribution de la colonne au barycentre, en Y, de l'événement.

$e_2$) une sous-étape de détermination :

- de l'énergie totale induite par l'événement dans l'ensemble des photodétecteurs,
- des coordonnées barycentriques $(X_1, Y_1)$ de l'événement par rapport aux N photodétecteurs.

8. Procédé selon l'une des revendications 1 à 7, comportant en outre, préalablement à l'étape b),une étape :

$a'_1$)de détermination de la position présumée d'un événement.

9. Procédé selon la revendication 8, comportant en outre une étape :

$a'_2$)de délimitation d'un sous-ensemble de N' photodétecteurs parmi l'ensemble de N photodétecteurs, autour de la position présumée de l'événement, seuls les signaux des N' photodétecteurs de ce sous-ensemble étant traités selon les étapes b, c, d et e.

10. Procédé selon l'une des revendications 1 à 9, les photodétecteurs étant des photomultiplicateurs d'une gamma-caméra.

11. Procédé d'imagerie en correction d'atténuation par transmission, mettant en oeuvre le procédé selon la revendication 10.

12. Procédé d'imagerie en PET en coïncidence mettant en oeuvre le procédé selon la revendication 10.

13. Dispositif pour la détermination de la position $P_1$ d'un événement dans un système de coordonnée X, Y, en particulier par rapport à un ensemble de N photodétecteurs, comportant :

a) des moyens (266, 268) pour numériser des signaux délivrés par chaque photodétecteur, et pour calculer une valeur $N_{i,j}$ représentative de l'énergie du signal délivré par chaque photodétecteur,
b) des moyens (76, 78) pour calculer une position brute $P_0$ de l'événement, dispositif **caractérisé en ce qu'**il comporte en outre,
c) des moyens (80, 130, 132) pour déterminer une distance $d_{i,j}$ de chacun des photodétecteurs par rapport à la position brute,
d) des moyens (80, 146, 152) pour calculer une valeur corrigée $N'_{i,j}$ de l'énergie délivrée par chaque photodétecteur, ces moyens (80, 146, 152) effectuant ce calcul selon la formule $N'_{i,j}=F'(N_{i,j})$, où F' est une fonction :

* qui réduit (respectivement : ne modifie pas) la valeur $N_{i,j}$ pour le photodétecteur correspondant à $P_0$ et pour un certain nombre $N_1$ de photodétecteurs autour de $P_0$,
* qui accroît, ou qui ne modifie pas (respectivement : accroît) la valeur $N_{i,j}$ pour un certain nombre de photodétecteurs $N_2$ situés autour des $N_1$ photodétecteurs précédents,
* qui tend vers 0 au-delà,

e) des moyens (82) pour calculer la position $P_1$ de l'événement, ces moyens étant commandés par les moyens pour calculer les valeurs corrigées

$$N'_{i,j}=F'(N_{i,j}).$$

**14.** Dispositif selon la revendication 13, les moyens pour calculer la position brute $P_0$ de l'événement étant des moyens pour réaliser le calcul des coordonnées barycentriques dudit événement.

**15.** Dispositif selon la revendication 13 ou 14, les moyens pour calculer la nouvelle position $P_1$ de l'événement étant des moyens pour réaliser un calcul barycentrique de cette nouvelle position.

**16.** Dispositif selon la revendication 13, comportant en outre des moyens :

d') pour modifier la valeur de la position $(XC_{i,j}, YC_{i,j})$ des photodétecteurs situés au bord du champ de photodétecteurs, en une position $(XC'_{i,j}, YC_{i,j})$ où au moins l'une des deux inégalités suivantes est satisfaite : $|XC'_{i,j}|>|XC_{i,j}|$, $|YC'_{i,j}|>|YC_{i,j}|$.

**17.** Dispositif selon l'une des revendications 13 à 16, comportant en outre :

- un ensemble de N photodétecteurs (60, 60-1, ..., 60-2) disposés en lignes et en colonnes,
- des moyens pour regrouper les photodétecteurs d'une même colonne sur un bus colonne (62),
- des moyens pour regrouper les bus colonnes sur une bus série (64).

**18.** Dispositif selon la revendication 17, un opérateur, regroupant les moyens b) à e), étant connecté sur le bus série, et réalisant un traitement série des données.

**19.** Dispositif selon la revendication 17, un opérateur, regroupant les moyens b) à e), étant connecté aux bus colonnes, et réalisant un traitement parallèle des données.

**20.** Dispositif selon la revendication 13, comportant des moyens (90, 90-1, ..., 90-6) pour déterminer, pour chaque colonne i :

- la contribution de la colonne à l'énergie totale induite par l'événement dans l'ensemble des photodétecteurs,
- la contribution de la colonne au barycentre, en X, de l'événement,
- la contribution de la colonne au barycentre, en Y de l'événement,

et des moyens (106, 110, 114) pour déterminer :

- l'énergie totale induite par l'événement dans l'ensemble des photodétecteurs,
- les coordonnées barycentriques $(X_0, Y_0)$ de l'événement par rapport aux N photodétecteurs.

**21.** Dispositif selon la revendication 15, comportant en outre des moyens pour :

b') déterminer, à l'aide des valeurs de signal pondéré $N'_{i,j}$, pour chaque colonne i :

- la contribution de la colonne à l'énergie totale induite par l'événement dans l'ensemble des photodétecteurs,
- la contribution de la colonne au barycentre, en X, de l'événement,
- la contribution de la colonne au barycentre, en Y, de l'événement.

c') déterminer, à l'aide des valeurs des contributions obtenues en b') :

- l'énergie totale induite par l'événement dans l'ensemble des photodétecteurs,
- des nouvelles coordonnées barycentriques $(X_1, Y_1)$ de l'événement par rapport aux N photodétecteurs.

**22.** Dispositif selon l'une des revendications 13 à 21, comportant en outre des moyens (70) pour détecter la position présumée d'un événement.

**23.** Dispositif selon la revendication 22, comportant en outre des moyens pour délimiter un sous-ensemble de $N_1$ photodétecteurs parmi l'ensemble de N photodétecteurs, autour de la position présumée de l'événement

**24.** Dispositif selon l'une des revendications 13 à 23, les photodétecteurs étant des photomultiplicateurs d'une gamma-caméra.

**Claims**

**1.** Process for determining the position ($P_1$) of an event in a coordinate system (X, Y) by means of a set of N photodetectors arranged in rows and columns, a random detector of a column of rank i and a row of rank j, being locatable in the coordinate system (X, Y), the event inducing a signal in the N photodetectors, the process involving the following steps:

a) digitizing the signal supplied by each photodetector and calculating a value $N_{i,j}$ representative of the energy supplied by each photodetector,
b) calculating, as a function of $N_{i,j}$ and the position of the photodetectors, an uncorrected position $P_0$ ($X_0$, $Y_0$) of the event relative to the set of photodetectors,
c) determination of the distance $D_{i,j}$ of each of the photodetectors relative to the position $P_0$,
the process being **characterized in that** it also comprises
d) the calculation of a corrected value $N'_{i,j} = F'(N_{i,j})$, in which F' is a function:

which reduces (respectively does not modify) the value $N_{i,j}$ for the photodetector corresponding to $P_0$ and for a certain number $N_1$ of photodetectors around $P_0$,
which increases or does not modify (respectively increases) the value $N_{i,j}$ for a certain number of photodetectors $N_2$ around the preceding $N_i$ photodetectors,
which tends towards 0 at higher values,

e) calculating the position $P_i$ of the event as a function of the position of the photodetectors and $N'_{i,j}$.

**2.** Process according to claim 1, in which the uncorrected position $P_0$ of the event is implemented by calculating the barycentric coordinates ($X_0$, $Y_0$) of said event as a function of $N_{i,j}$ and the position ($XC_{i,j}$, $YC_{i,j}$) of each photodetector.

**3.** Process according to claim 1 or 2, in which the position $P_1$ of the event is implemented by calculating the barycentric coordinates ($X_1$, $Y_1$) of the event as a function of $N'_{i,j}$ and the position ($XC_{i,j}$, $YC_{i,j}$) of each photodetector.

**4.** Process according to one of claims 1 to 3, comprising the following step before step e), for each photodetector at the edge of the field of N photodetectors:

$e_0$) modify the value of the position ($XC_{i,j}$, $YC_{i,j}$) the new pair of values ($XC'_{i,j}$, $YC'_{i,j}$) being such that either the $|XC'_{i,j}| > |XC_{i,j}|$ relation or the $|YC'_{i,j}| > |YC_{i,j}|$ relation is satisfied, or both of these relations are satisfied.

**5.** Process according to one of claims 2 and 3, the barycentric calculations being carried out in sequence.

**6.** Process according to claim 2, in which the barycentric coordinates ($X_0$, $Y_0$) of the event could be determined using the following sub-steps:

$b_1$) a sub-step in which the following are determined for each column i:

- the contribution of the column to the total energy induced by the event in the set of photodetectors,
- the contribution of the column to the X value of the barycentre of the event,
- the contribution of the column to the Y value of the barycentre of the event,

$b_2$) a sub-step in which the following are determined:

- the total energy induced by the event in the set of photo-detectors,
- the barycentric coordinates ($X_0$, $Y_0$) of the event with respect to the N photo-detectors.

**7.** Process according to claim 3, in which the barycentric coordinates ($X_1$, $Y_1$) of the event could be determined using the following sub-steps:

$e_1$) a sub-step in which the following are determined for each column i:

- the contribution of the column to the total energy induced by the event in the set of photodetectors,
- the contribution of the column to the X value of the barycentre of the event,
- the contribution of the column to the Y value of the barycentre of the event,

$e_2$) a sub-step in which the following are determined:

- the total energy induced by the event in the set of photodetectors,
- the barycentric coordinates ($X_1$, $Y_1$) of the event with respect to the N photodetectors.

8. Process according to one of claims 1 to 7, also comprising an additional step prior to step b):

$a'_1$) to determine the presumed position of an event.

9. Process according to claim 8, also comprising a step:

$a'_2$) to delimit a subset of N' photodetectors among the set of N photodetectors around the presumed position of the event, only the signals from the N' photodetectors in this subset being processed according to steps b, c, d and e.

10. Process according to one of claims 1 to 9, the photodetectors being photomultipliers of a gamma-camera.

11. Imagery process in correction of transmission attenuation, embodying the process according to claim 10.

12. PET coincidence imagery process embodying the process according to claim 10.

13. Device for determining the position of an event in a coordinate system X, Y, particularly with respect to set of N photodetectors, comprising:

a) means (266, 268) of digitizing signals output by each photodetector, and of calculating a value $N_{i,j}$ representing the energy of the signal output by each photodetector,
b) means (76, 78) of calculating an uncorrected position $P_0$ of the event, **characterized in that** it also comprises:
c) means (80, 130, 132) of determining a distance $d_{i,j}$ of each photodetector relative to the uncorrected position,.
d) means (80, 146, 152) for calculating a corrected value $N'_{i,j}$ of the energy supplied by each photodetector, said means (80, 146, 152) performing the said calculation in accordance with the formula $N'_{i,j} = F'(N_{i,j})$, in which F' is a function:

- which reduces (respectively does not modify) the value $N_{i,j}$ for the photodetector corresponding to $P_0$ and for a certain number $N_i$ of photodetectors around $P_0$,
- which increases or which does not modify (respectively increases) the value $N_{i,j}$ for a certain number of photodetectors $N_2$ around said preceding $N_1$ photodetectors,
- - which tends towards 0 for higher values,

e) means (82) for calculating the position $P_1$ of the event, said means being controlled by the means for calculating the corrected values $N'_{i,j} = F' (N_{i,j})$.

14. Device according to claim 13, the means of calculating the uncorrected position $P_0$ of the event being means of calculating the barycentric coordinates of the said event.

15. Device according to claim 13 or 14, the means of calculating the new position $P_1$ of the event, being the means for implementing a barycentric calculation of this new position.

16. Device according to claim 13, also comprising means:

d') of modifying the value of the position ($XC_{i,j}$, $YC_{i,j}$) of the photodetectors located at the edge of the photodetectors field, to a position (XC'i,j, YCi,j) in which the [XC'i.j] > ]XCi,j] relation or the ]YC'i,j] > [YCi,j] relation is satisfied, or both of these relations are satisfied.

17. Device according to one of claims 13 to 16, also comprising:

- a set of N photodetectors (60, 60-1, ..., 60-2) laid out in rows and columns,
- means of grouping the photodetectors in the same column on a column bus (62),
- means of grouping the column buses on a serial bus (64).

18. Device according to claim 17, an operator including all means b) to e) being connected to the serial bus and performing serial processing of the data.

19. Device according to claim 17, an operator grouping means b) to e) being connected to the serial bus and implementing a parallel data processing.

20. Device according to claim 13, comprising means (90, 90-1, ..., 90-6) of determining the following for each column i:

- the contribution of the column to the total energy induced by the event in the set of photodetectors,
- the contribution of the column to the X value of the barycentre of the event,
- the contribution of the column to the Y value of the barycentre of the event,
    and means (106, 110, 114) in which the following are determined:

- the total energy induced by the event in the set of photodetectors,
- the barycentric coordinates (Xo, Yo) of the event with respect to the N photodetectors.

21. Device according to claim 15, also comprising means for:

b') determining the following for each column i making use of the values of the weighted signal N'i,j:

- the contribution of the column to the total energy induced by the event in the set of photodetectors.
- the contribution of the column to the X value of the barycentre of the event,
- the contribution of the column to the Y value of the barycentre of the event,

c') determining the following making use of the values of the contributions obtained in b')
- the total energy induced by the event in the set of photodetectors
- the new barycentric coordinates (X1, Y1) of the event with respect to the N photodetectors.

22. Device according to any one of claims 13 to 21, also comprising means (70) of detecting the presumed position of an event.

23. Device according to claim 22, also comprising means of delimiting a subset of $N_1$ photodetectors among the set of N photodetectors around the presumed position of the event.

24. Device according to one of claims 13 to 23, the photodetectors being the photomultipliers in a gamma-camera.

**Patentansprüche**

1. Verfahren zur Bestimmung der Position $P_1$ eines Ereignisses in einem X-Y-Koordinatensystem mittels einer Gruppe von N zeilen- und spaltenförmig angeordneten Photodetektoren, wobei jeder beliebige Detektor einer Spalte des Rangs i und einer Zeile des Rangs j in dem X-Y-Koordinatensystem lokalisierbar ist, das Ereignis in den N Photodetektoren ein Signal induziert und das Verfahren die folgenden Schritte umfasst:

a) Nummerierung des von jedem Photodetektor gelieferten Signals und Berechnung eines Werts $N_{i,j}$, der repräsentativ ist für die durch jeden Photodetektor gelieferte Energie,
b) Berechnung einer Rohposition $P_0$ ($X_0$, $Y_0$) des Ereignisses aufgrund der $N_{i,j}$-Werte und der Position der Photodetektoren, bezogen auf die Gesamtheit der Photodetektoren,
c) Bestimmung des Abstands $d_{i,j}$ jedes der Photodetektoren in Bezug auf die Position $P_0$, wobei das Verfahren **dadurch gekennzeichnet ist, dass** es außerdem umfasst:

d) die Berechnung eines korrigierten Werts $N'_{i,j} = F'(N_{i,j})$, wo F' eine Funktion ist:

* die den Wert $N_{i,j}$ für den $P_0$ entsprechenden Photodetektor und für eine bestimmte Anzahl $N_1$ von Photodetektoren im Umkreis von $P_0$ reduziert (beziehungsweise: nicht modifiziert),
* die den Wert $N_{i,j}$ für eine bestimmte Anzahl $N_2$ von Photodetektoren im Umkreis der vorhergehenden Photodetektoren $N_1$ erhöht, oder nicht modifiziert (beziehungsweise erhöht),
* die darüber hinaus gegen 0 tendiert,

e) Berechnung der Position $P_1$ des Ereignisses in Abhängigkeit von der Position der Photodetektoren und der $N'_{i,j}$-Werte.

2. Verfahren nach Anspruch 1, bei dem die Berechnung der Rohposition $P_0$ des Ereignisses realisiert wird, indem man die Schwerpunktkoordinaten $(X_0, Y_0)$ des genannten Ereignisses in Abhängigkeit von $N_{i,j}$ und der Position $(XC_{i,j}, YC_{i,j})$ jedes Photodetektors berechnet.

3. Verfahren nach Anspruch 1 oder 2, bei dem die Berechnung der Position $P_1$ des Ereignisses realisiert wird, indem man die Schwerpunktkoordinaten $(X_1, Y_1)$ des genannten Ereignisses in Abhängigkeit von $N'_{i,j}$ und der Position $(XC_{i,j}, YC_{i,j})$ jedes Photodetektors berechnet.

4. Verfahren nach einem der Ansprüche 1 bis 3, das vor dem Schritt e) und für die am Rande des Feldes von N Photodetektoren befindlichen Photodetektoren folgenden Schritt umfasst:

$e_0$) Modifizierung des Werts der Position $(XC_{i,j}, YC_{i,j})$, wobei das neue Wertepaar $(XC'_{i,j}, YC'_{i,j})$ derartig ist, dass wenigstens eine der beiden folgenden Ungleichheiten bzw. Ungleichungen befriedigt wird: $|XC'_{i,j}| < |XC_{i,j}|$, $|YC'_{i,j}| < |YC_{i,j}|$.

5. Verfahren nach einem der Ansprüche 2 oder 3, wobei die Schwerpunktberechnungen auf sequentielle Weise durchgeführt werden.

6. Verfahren nach Anspruch 2, wobei die Bestimmung der Schwerpunktkoordinaten $(X_0, Y_0)$ des Ereignisses die folgenden Teilschritte umfasst:

$b_1$) einen Teilschritt zur Bestimmung - für jede Spalte i:

- des Beitrags der Spalte zur der durch das Ereignis in der Gesamtheit der Photodetektoren induzierten Gesamtenergie,
- des Beitrags der Spalte zum Schwerpunkt des Ereignisses in X-Richtung,
- des Beitrags der Spalte zum Schwerpunkt des Ereignisses in Y-Richtung;

$b_2$) einen Teilschritt zur Bestimmung:

- der durch das Ereignis in der Gesamtheit der Photodetektoren induzierten Gesamtenergie,
- der Schwerpunktkoordinaten $(X_0, Y_0)$ des Ereignisses in Bezug auf die N Photodetektoren.

7. Verfahren nach Anspruch 3, wobei die Bestimmung der Schwerpunktkoordinaten $(X_1, Y_1)$ des Ereignisses die folgenden Teilschritte umfasst:

$e_1$) einen Teilschritt zur Bestimmung - für jede Spalte i:

- des Beitrags der Spalte zur der durch das Ereignis in der Gesamtheit der Photodetektoren induzierten Gesamtenergie,
- des Beitrags der Spalte zum Schwerpunkt des Ereignisses in X-Richtung,
- des Beitrags der Spalte zum Schwerpunkt des Ereignisses in Y-Richtung.

$e_2$) einen Teilschritt zur Bestimmung:

- der durch das Ereignis in der Gesamtheit der Photodetektoren induzierten Gesamtenergie,
- der Schwerpunktkoordinaten $(X_1, Y_1)$ des Ereignisses in Bezug auf die N Photodetektoren.

**8.** Verfahren nach einem der Ansprüche 1 bis 7, das außerdem vor dem Schritt b) folgenden Schritt umfasst:

$a'_1$) Bestimmung der vermuteten Position eines Ereignisses.

**9.** Verfahren nach Anspruch 8, das außerdem folgenden Schritt umfasst:

$a'_2$) Abgrenzung einer Teilgruppe von N' Photodetektoren aus der Gesamtheit von N Photodetektoren im Umkreis der angenommenen Position des Ereignisses, wobei nur die Signale der N' Photodetektoren dieser Teilgruppe nach den Schritten b, c, d und e verarbeitet werden.

**10.** Verfahren nach einem der Ansprüche 1 bis 9, wobei die Photodetektoren Photovervielfacher einer Gamma-Kamera sind.

**11.** Dämpfungskorrektur-Bilderzeugungsverfahren durch Transmission, bei dem das Verfahren nach Anspruch 10 angewandt wird.

**12.** Koinzidenz-PET-Bilderzeugungsverfahren, bei dem das Verfahren nach Anspruch 10 angewandt wird.

**13.** Einrichtung zur Bestimmung der Position $P_1$ eines Ereignisses in einem X-Y-Koordinatensystem, insbesondere in Bezug auf eine Gruppe von N Photodetektoren, umfassend:

a) Einrichtungen (266, 268) zum Digitalisieren der durch jeden Photodetektor gelieferten Signale und zum Berechnen eines Wertes $N_{i,j}$, der repräsentativ ist für die Energie des durch jeden Photodetektor gelieferten Signals,

b) Einrichtungen (76, 78) zum Berechnen einer Rohposition $P_0$ des Ereignisses, wobei diese Einrichtung **dadurch gekennzeichnet ist, dass** sie umfasst:

c) Einrichtungen (80, 130, 132) zum Bestimmen eines Abstands $d_{i,j}$ jedes der Photodetektoren in Bezug auf die Rohposition,

d) Einrichtungen (80, 146, 152) zum Berechnen eines korrigierten Wertes $N'_{i,j}$ der durch jeden Photodetektor gelieferten Energie, wobei diese Einrichtungen (80, 146, 152) diese Berechnung nach der Formel $N'_{i,j} = F'(N_{i,j})$ durchführen, wo F eine Funktion ist:

* die den Wert $N_{i,j}$ für den $P_0$ entsprechenden Photodetektor und für eine bestimmte Anzahl $N_1$ von Photodetektoren im Umkreis von $P_0$ reduziert (beziehungsweise: nicht modifiziert),
* die den Wert $N_{i,j}$ für eine bestimmte Anzahl $N_2$ von Photodetektoren im Umkreis der vorhergehenden Photodetektoren $N_1$ erhöht, oder nicht modifiziert (beziehungsweise erhöht),
* die darüber hinaus gegen 0 tendiert,

e) Einrichtungen (82) zur Berechnung der Position $P_1$ des Ereignisses, wobei diese Einrichtungen gesteuert werden durch die Einrichtungen zur Berechnung der korrigierten Werte $N'_{i,j} = F'(N_{i,j})$.

**14.** Vorrichtung nach Anspruch 13, wobei die Einrichtungen zum Berechnen der Rohposition $P_0$ des Ereignisses Einrichtungen zum Durchführen der Berechnung der Schwerpunkt-Koordinaten des genannten Ereignisses sind.

**15.** Vorrichtung nach Anspruch 13 oder 14, wobei die Einrichtungen zum Berechnen der neuen Position $P_1$ des Ereignisses Einrichtungen zum Durchführen einer Schwerpunktberechnung dieser neuen Position sind.

**16.** Vorrichtung nach Anspruch 13, die außerdem Einrichtungen umfasst:

d') um den Wert der Position ($XC_{i,j}$, $YC_{i,j}$) der Photodetektoren zu modifizieren, die sich am Rand des Photodetektorenfeldes in einer Position ($XC'_{i,j}$, $YC'_{i,j}$) befinden, wo wenigstens eine der beiden folgenden Ungleichheiten bzw. Ungleichungen befriedigt wird: $|XC'_{i,j}| < |XC_{i,j}|$, $|YC'_{i,j}| < |YC_{i,j}|$.

**17.** Vorrichtung nach einem der Ansprüche 13 bis 16, die außerdem umfasst:

- eine Gruppe von N zeilen- und spaltenförmig angeordneten Photodetektoren (60, 61-1, ..., 60-2),
- Einrichtungen zum Zusammenfassen der Photodetektoren einer selben Spalte auf einem Spaltenbus (62),
- Einrichtungen zum Zusammenfassen der Spaltenbusse auf einem Serienbus (64).

18. Vorrichtung nach Anspruch 17, wobei ein Operator, der die Einrichtungen b) bis e) zusammenfasst, an den Serienbus angeschlossen ist und eine Serienverarbeitung der Daten durchführt.

19. Vorrichtung nach Anspruch 17, wobei ein Operator, der die Einrichtungen b) bis e) zusammenfasst, an den Spaltenbus angeschlossen ist und eine Parallelverarbeitung der Daten durchführt.

20. Vorrichtung nach Anspruch 13, die Einrichtungen (90, 90-1, ..., 90-6) umfasst, um für jede Spalte zu bestimmen:

   - den Beitrag der Spalte zur der durch das Ereignis in der Gesamtheit der Photodetektoren induzierten Gesamtenergie,
   - den Beitrag der Spalte zum Schwerpunkt des Ereignisses in X-Richtung,
   - den Beitrag der Spalte zum Schwerpunkt des Ereignisses in Y-Richtung,

   und Einrichtungen (106, 110, 114) zur Bestimmung:

   - der durch das Ereignis in der Gesamtheit der Photodetektoren induzierten Gesamtenergie,
   - der Schwerpunktkoordinaten $(X_0, Y_0)$ des Ereignisses in Bezug auf die N Photodetektoren.

21. Vorrichtung nach Anspruch 15, die außerdem Einrichtungen umfasst, um zu bestimmen:

   b') mit Hilfe der Werte des gewichteten Signals $N'_{i,j}$ für jede Spalte i:

   - den Beitrag der Spalte zur der durch das Ereignis in der Gesamtheit der Photodetektoren induzierten Gesamtenergie,
   - den Beitrag der Spalte zum Schwerpunkt des Ereignisses in X-Richtung,
   - den Beitrag der Spalte zum Schwerpunkt des Ereignisses in Y-Richtung.

   c') mit Hilfe der in b') erhaltenen Werte der Beiträge:

   - die durch das Ereignis in der Gesamtheit der Photodetektoren induzierte Gesamtenergie,
   - neue Schwerpunktkoordinaten $(X_1, Y_1)$ des Ereignisses in Bezug auf die N Photodetektoren.

22. Vorrichtung nach einem der Ansprüche 13 bis 21, die außerdem Einrichtungen (70) zum Detektieren der angenommenen Position eines Ereignisses umfasst.

23. Vorrichtung nach Anspruch 22, die außerdem Einrichtungen zur Abgrenzung einer Teilgruppe von $N_1$ Photodetektoren aus der Gesamtheit von N Photodetektoren umfasst, um die angenommene Position des Ereignisses herum.

24. Vorrichtung nach einem der Ansprüche 13 bis 23, wobei die Photodetektoren Photovervielfacher einer Gamma-Kamera sind.

FIG. 1

FIG. 2B

FIG. 2A

EP 0 950 197 B1

FIG. 3

FIG. 4

FIG. 5 A

FIG. 5 B

$N_{ij}$     $XC_{ij}$     $YC_{ij}$

78

122

120

$N'_{ij}$     $XC_{ij}$     $YC_{ij}$

80

82

$X_1, Y_1$

$X_1, Y_1$

88

FIG. 6

FIG. 7

FIG. 8

FIG. 9

FIG. 10

FIG. 11A

FIG. 11B

FIG. 12